# EUROPEAN PATENT APPLICATION

(11) **EP 1 231 263 A1**
(43) Date of publication of application: **14.08.2002**
(21) Application number: 01400341.2
(22) Date of filing: 09.02.2001
(51) Int. Cl.: C12N 5/08, A61K 35/12

(54) **Multipotent O-2A progenitors from the neurohypophysis**

(71) Applicant: CENTRE NATIONAL DE LA RECHERCHE SCIENTIFIQUE (CNRS), 75794 Paris Cedex 16 (FR)
(72) Inventor: Rougon, Geneviève, Bat Le Thais, 13009 Marseille (FR); Coquillat, Delphine, 13006 Marseille (FR); Dubrec, Pascale, 13009 Marseille (FR)
(74) Representative: Warcoin, Jacques

(57) **Abstract**

An isolated population of mammalian neurohypophysis cells comprising multipotent cells has been identified. O-2A progenitor cells are described which are able to differentiate into at least oligodendrocytes and/or type II astrocytes and/or neurons. The invention includes also methods for isolating, culturing, transplanting such cellular population, and their use as a medicament for a neural disorder or a neural disease.

## Description

This invention relates to neurobiology and specifically to the isolation and use of population of multipotent stem cells isolated from the neurohypophysis of mammals. More specifically the invention relates to oligodendrocyte-type 2 astrocyte (O-2A) progenitor cells.
The invention relates also to methods using this population in particular for regeneration and use of mammalian multipotent O-2A progenitor cells and their progeny for therapeutic treatments and cell cultures.

It is thought that multipotent progenitor cells could be very useful in the treatment of disorders associated to a lack of or a loss or abnormal activity of fully differentiated cells of an organ. There is thus a need for finding multipotent cells sources. There are many tissues that can be investigated in order to isolate such progenitor cells, but the isolation methods are specific from each source of progenitor cells.
In particular, for the central nervous system (CNS), a population of multipotent progenitor cells, called oligodendrocyte - type II astrocyte progenitor cells (O-2A) has already been identified. This progenitor population has been shown to be a bifurcation point in cell lineage and cellular differentiation. Studies by Raff and colleagues (Raff, et al., Nature, 303:390, 1983) showed the existence of a bipotential glial progenitor cell in the rat optic nerve which, *in vitro* under the appropriate growth conditions, has the power to differentiate into oligodendrocytes or type II astrocytes and under certain conditions to neurons (Kondo and Raff, 2000). O-2A progenitors from other neural regions have been shown to be bipotential *in vitro,* giving rise either to oligodendrocytes or type 2 astrocytes depending on the culture medium (Butt (1998) and Levine (1987)).
Cells of the CNS are classified as either neurons or glial cells. Glial cells can be further divided into oligodendrocytes and astrocytes. Oligodendrocytes are the myelin producing cells of the CNS. Death of oligodendrocytes appears to induce the demyelination seen in multiple sclerosis (Waxman, S. G., New Engl. J Med., 306:1529, 1982), or periventricular white matter injury thought to underlie spastic motor and cognitive deficits frequently seen in premature infants (Oka, et al., J Neurosci., 13:1441, 1993).

Therapeutic grafting has particular promise in demyelinating disease such as multiple sclerosis. In the rat, oligodendrocyte progenitor cultures that have been grown and expanded in vitro can be engrafted back into the animal. Mice mutant for myelin production can serve as the recipients of these cells, and marked cells can be seen to migrate, engraft, differentiate and myelinate recipient nerve fibers (Espinosa de los Monteros, et al., Dev. Neurosci., 14:98, 1992). Such observations suggest of the use of human oligodendrocyte progenitors in grafting, as a therapy in demyelinating disease, and perhaps following trauma to the CNS. Further, in recent years the idea of grafting human tissue as a therapy for neurodegenerative disease has received increased attention (Bjorkland, Nature, 362:414, 1993).
Isolation and clonal propogation of O-2A progenitors and progeny has also been obtained from neural crest, as described in US 5,693,482.
There is still a need to identify progenitor cells sources which are syngenic, rapidly expandable and successfully implanted in neural tissue, namely the brain.
An object of the invention is to identify other mammalian sources of O-2A progenitors capable of being successfully grafted in order to give rise in vivo to cells allowing to compensate a neural disorder.
Another object is to identify mammalian tissues capable of being successfully grafted in order to give rise in vivo to neurons.
An other object is, considering that a transplantation approach can be limited by the availability of donor tissue, to provide a source of progenitor cells useful for treating neurological diseases of the CNS and the PNS in model animal systems and in human. There is also a need to obtain cultures of O-2A progenitors and cells derived from O-2A progenitors which can be produced at a large scale, subcultured over time, used for assaying the effects of various neuroactive compositions on these cells for neurobiological and neuropharmaceutical studies and CNS drug discovery efforts, as well as therapy.
A further object is to optimize the conditions of isolation and culture of such progenitor cells, considering the extreme complexity of the systems. Indeed, there are numerous types of cells in the CNS and many different neurotrophic factors which influence their growth and differentiation. Depending on the type of cell and the region of the brain in which the cell resides, a different neurotrophic factor or specific combination of factors affect the survival, proliferation and differentiation of the cell in vivo. Each type of cell responds to different combinations of neurotransmitters, neurotrophic factors, and other molecules in its natural environment.
A further object is to provide genetically engineered O-2A progenitors and their progeny cells containing genes of interest.
A further object is to provide methods for identifying markers in vivo characterizing O-2A progenitors and their progeny.

In accordance with the objects cited the invention relates according to a first aspect to an isolated population of mammalian neurohypophysis (NH) cells comprising multipotent cells.
Said multipotent cells are able to differentiate into at least oligodendrocytes and/or astrocytes and/or neurons.
According to an embodiment said population is derived from newborn neurohypophysis when this structure is still developing or from adult neurohypophysis.
According to an embodiment said population is derived from adult neurohypophysis after a physiological stimulus.
According to an embodiment said population is an explant of neurohypophysis, cultivated in an approriate culture medium.
The invention relates also to a method for obtaining said cellular, comprising the steps of preparing a suspension from a neurohypophysis explant, culturing the suspension in an appropriate medium for growth and/or proliferation of said population.
The invention relates also to a method for isolating said cellular population, comprising
- culturing a neurohypophysis explant wherein said explant is maintainable in culture and includes progenitor cells that have the ability to differentiate into at least oligodendrocytes and/or type II astrocytes and/or neurons ;
- contacting said explant with an agent which causes proliferation of progenitor cells of said explant;
- isolating from said explant progenitor cells that proliferate in response to said agent.
The invention relates also to a method for obtaining O-2A progenitor cells and/or their progeny comprising
- culturing a neurohypophyse explant wherein said explant is maintainable in culture and includes progenitor cells that have the ability to differentiate ;
- contacting said explant with an agent which causes proliferation of progenitor cells of said explant and differentiation into oligodendrocyte and/or type 2 astrocyte ;
- contacting said suspension with markers for O-2A progenitor cells and/or their progeny;
- isolating O-2A progenitor cells and/or their progeny.
The isolation technique of O-2A progenitor cells and/or their progeny may include for instance one of magnetic separation, antibody coated magnetic beads, affinity chromatography, antibodies attached to a matrix, responsiveness to growth factors, specific gene expression, antigenic cell specific surface markers, basic morphology.
The method may further comprise the preparation of an isolated cellular composition containing at least 50 %, of neurohypopysis O-2A progenitor cells and/or their progeny.
The invention relates also to a method for screening compounds having an ability to modulate one of growth, proliferation and/or differentiation of progenitor cells obtained from neurohypophyse comprising :
- culturing a neurohypophysis explant wherein said explant is maintainable in culture and includes progenitor cells that have the ability to differentiate,
- contacting said explant with an agent which causes proliferation of progenitor cells of said explant,
- contacting said explant with a tested compound,
- detecting effect on one of growth, proliferation and/or differentiation of progenitor cells by comparing the results without the compound.
The invention relates also to a method for obtaining an isolated population of transformed mammalian multipotent oligodendrocytes-type 2 astrocytes (O-2A) progenitor cells and/or their progeny, said method comprising the introduction of at least a nucleic acid. The nucleic acid may be homologous or heterologous. The transformation may be namely genetic engineering.
The nucleic acid may be a gene or a fragment of gene of interest, which introduction in O-2A progenitors and/or their progeny will correct an abnormal function or induce a new characteristic. The nucleic acid will be for instance a therapeutic gene, a gene for neurotrophic or survival factor, a gene for migratory factor, an immortalizing oncogene, a marker gene such as beta galactosidase gene, a selective marker allowing to identify an ability to grow under a selective pressure.
The invention relates also to a population of transformed mammalian multipotent O-2A progenitor cells and/or their progeny obtainable by said method.
The invention relates also to a method for providing neurophypophisis O-2A progenitor cells and/or their progeny in at least one location of the brain, comprising the transplantation of O-2A progenitor cells in the brain. It has indeed be shown that neurohypophysis O-2A progenitors have a migratory capacity in the brain in particular, thereby O-2A progenitors are capable of traveling from a first location where the cells are implanted, to at least a second location where they may differentiate.
The invention relates also to this method wherein the O-2A progenitor cells have been prior transformed.
The invention relates also to a method to assay in vivo development and differentiation of a neurohypophysis explants containing O-2A progenitor cells, said method comprising :
- extracting a newborn or adult neurohypophysis explant;
- labeling and transplantating the explant in the periventricular zone of newborn or adult mammalian brain;
- identifying the distribution of transplanted cells in the brain.
The invention relates also to a method for screening antibodies capable of recognizing surface markers which characterize multipotent O-2A progenitor cells and/or their progeny, comprising culturing said cells in an appropriate medium, adding tested antibodies, identifying the complex antibody-marker.
The invention relates also to a method for providing antibodies capable of recognizing surface markers which characterize multipotent O-2A progenitor cells and/or their progeny comprising immunizing an animal with said cells, isolating the antibodies produced.
The invention relates also to an isolated mammalian progenitor cell wherein said progenitor cell is extracted from neurohypophysis and is able to differentiate into at least oligodendrocyte and/or astrocyte and/or neuron.
The invention relates also to a method for obtaining at least one of said isolated cell comprising :
- culturing a neurohypophyse explant wherein said explant is maintainable in culture and includes progenitor cells that have the ability to differentiate into at least oligodendrocytes and type II astrocytes ;
- isolating from said explant at least one progenitor cell that have the ability to differentiate into at least oligodendrocytes and type II astrocytes.
The invention relates also to a pharmaceutical composition comprising a population of neurohypophysis O-2A progenitor cells and/or their progeny, or comprising said population that has been transformed, and a vehicle pharmaceutically acceptable.
The invention relates also to this composition comprising, as a cellular population, at least 50 % of the O-2A progenitor cells and/or their progeny.
The invention relates also to such composition used as a medicament for a neural disorder or a neural disease.
The invention relates also to the use of a population obtained by the method previously mentioned for the preparation of a pharmaceutical composition for treatment of a neural disorder or a neural disease.
The invention relates also to the use of an explant of neurohypophysis for the preparation of a graft for treatment of a neural disorder or a neural disease.
The neural disease may be in particular Alzheimer's disease, Parkinson's disease.

Other features and advantages of the invention will be apparent from the following detailed description, illustrated by the drawings.
Colours refer to staining with markers and fluorescent compounds allowing a better view of the cell types.

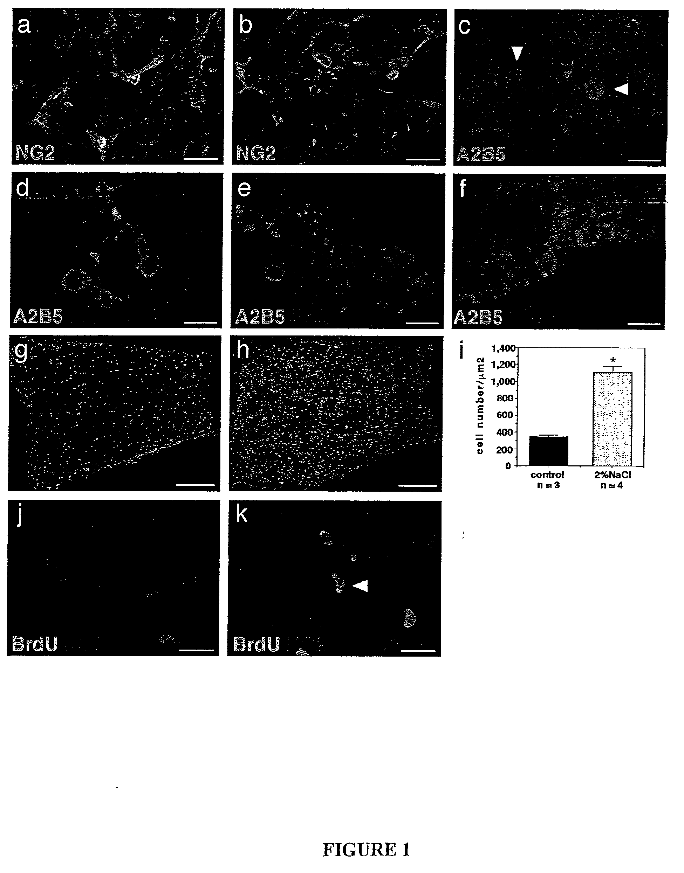

### Figure 1 shows that O-2A progenitors are NH-resident cells

Immunohistochemistry on adult NH sections was performed to identify the resident cell populations. Pituicytes are recognized by labeling with an anti-vimentin (a in red) or anti-S-100 (b in red) antibody. Double-staining using an anti-NG2 antibody (a marker for O-2A cells) allows the identification of a population of precursors (a and b in green) distinct from pituicytes. The TOPRO3 marker is used to identify cell nuclei (a and b in blue). A2B5 labeling of a whole mount preparation shows the presence of precursors in newborn (d in green) and adult (c and e in green) control animals and in the adult dehydrated (f in green) rats. Double-labeling with an anti-BrdU antibody shows that those precursors proliferate during development (d in red) and in the adult after dehydration (f in red) while no BrdU labeling is observed in adult control rats (e in red)

Double labeling on sections using anti-BrdU and anti-NG2 showed that NG2+ cells proliferate in adult rats after dehydration (k) but not in adult rat control (j). Proliferation is analyzed by BrdU incorporation and immunohistochemistry using an anti-BrdU antibody. NH preparations derived from adult control (g) or dehydrated (h) rats show a three-fold increase in proliferation after dehydration (i) (1111+/-45 BrdU+ cells per µm2 in dehydrated compared to 322+/-20 BrdU+ cells per µm2 in control animal). Scale bars : a-b, j-k ,13 µm ; c-f, 10 µm ; g-h, 325 µm.

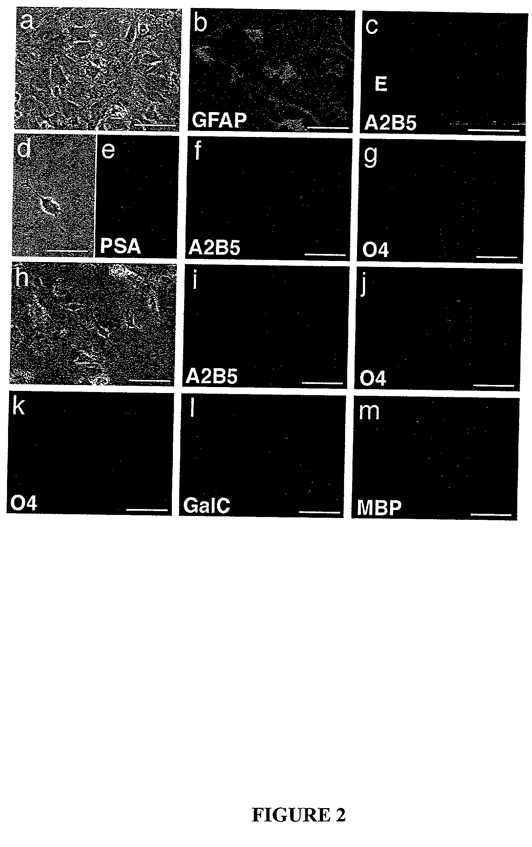

### Figure 2 shows the in vitro characterization of O-2A progenitors from the adult rat NH

Bipolar cells migrating out of the adult NH explants in vitro (a) are immunopositive for A2B5 (b) identifying them as O-2A progenitors. When BrdU, able to be incorporated by dividing cells, is injected into control (c) or dehydrated (d) adult rats prior to the explantations double positive A2B5+ (red) /BrdU+ (green) cells are observed in the outgrowth of explants derived from physiologically stimulated (dehydrated) rats (d) but these are never found in the control condition (c). E: explant. Scale bar, 58 µm.

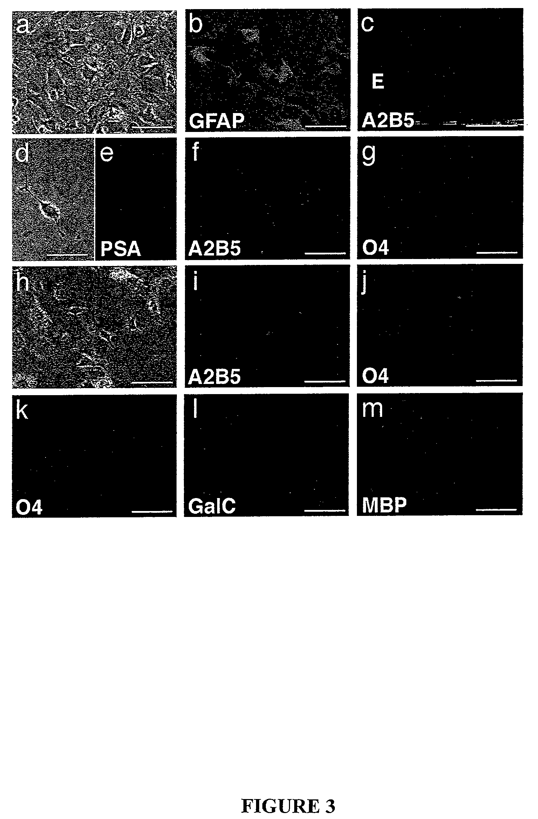

### Figure 3 shows the in vitro bipotentiality of O-2A progenitors from neonatal rat NH

When serum is included in the culture medium an extensive proliferation of fibroblasts and endothelial cells is observed around the explant (a). Immunofluorescence labeling shows that the majority of cells on the top of this monolayer are GFAP+ thus astrocytes (b). In defined medium, bipolar A2B5+ cells are clearly identified migrating out of the explant (c). Differentiation toward the oligodendrocyte lineage was followed by analysis of the expression of stage-specific markers. Twenty four hours after explantation, the migrating bipolar cells (d) are immunoreactive for both PSA-NCAM (e) and A2B5 (f), a few cells express the 04 antigen (g). Forty-eight hours after explantation, the majority of cells are multipolar, A2B5+ (h and i) and 04+ (j). After 14 days, cells are immunopositive for the mature oligodendrocyte markers, 04 (k) and GalC (1) and they show MBP labeling (m). E: explant. Scale bars: a-c, f-m, 58 µm; d-e, 37 µm.

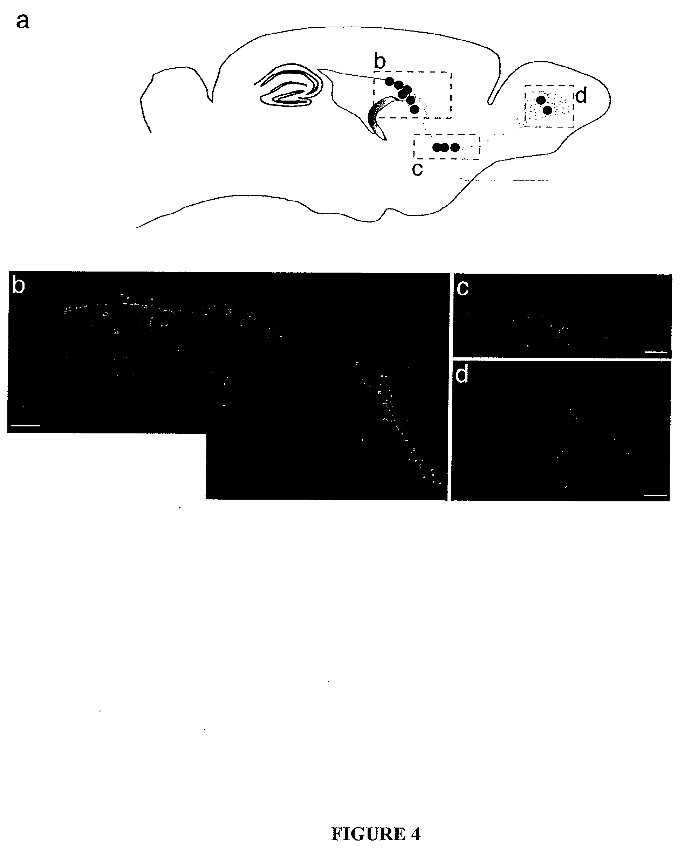

### Figure 4 shows the migration of NH derived cells in host mouse forebrain

Pieces of newborn hypophysis were labeled with Hoechst to identify their nuclei and implanted in mouse. Schematic saggital representation (a) of the host brain number 11 (table I) showing the repartition of Hoechst positive cells 21 days after transplantation. Areas including Hoechst+ cells are schematized by blue dots. Dotted rectangles in part (a) represent the areas in (b), (c) and (d) showing, in section, the presence of Hoechst+ cells in the subventricular zone, the rostral migratory stream and the olfactory bulb, respectively. Scale bars: b, 350 µm; c-d, 175 µm. The graft contained cells able to survive and migrate in the host brain.

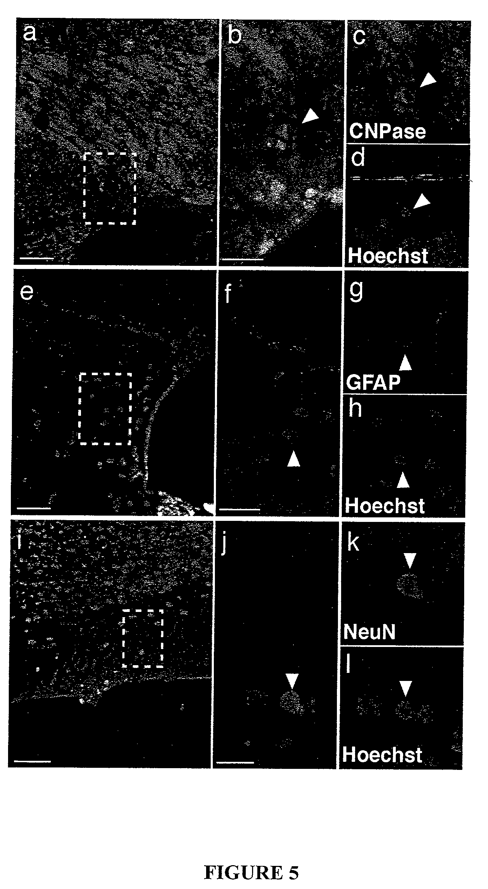

### Figure 5 shows the differentiation potential of newborn mice NH derived cells in vivo

Immunofluorescence analysis on sections of host brains was performed 21 days after transplantation of NH-derived cells- in the cerebral periventricular area of newborn mice to identify the phenotype.
These sections contain Hoechst+ cells (in blue), cells labeled with the anti-CNPase (a-c in green), -GFAP (e-g in red) or -NeuN (i-k in green) antibodies. Examples were selected from the host brain number 9 (table I) in the corpus callosum (a-d) and in the fimbriae (e-h) and from the host brain number 7 (table I) in the corpus callosum (i-l). Arrow heads indicate double positive cells. Scale bars : a, e, i, 83 µm; b-d, f-h, j-l, 33 µm.

The invention will now be described in more details.

The term progenitor cell (or stem cell, term also currently used) refers to an undifferentiated cell which is able to proliferate and to give rise to more progenitor cells having the ability to generate a high number of cells that differentiate into fully differentiated cells, that are called their progeny, exhibiting specialized characters. A neurohypohysis O-2A progenitor cells refers to a progenitor cell population arising in the neural lobe of the neurohypophysis and giving rise to a differentiated progeny, namely mature functional oligodendrocytes and type 2 astrocytes. Indifferently « O-2A progenitors » or « O-2A progenitor cells » will be used.
The term explant refers to a portin of an organ, the neurohypohisis, taken from the mammalian body and grown in an artificial medium.
The term isolated population of cell means that the cell is extracted from the body. For instance dissection of neural lobe of neurohypophysis is an isolation technique. The step further to isolation is purification in order to obtain a preparation that contains at least 75 % of the cells.
The term culture medium refers to a preparation for the culture of living cells. A tissue culture refers to the maintenance or growth of tissue, namely an explant, in vitro so as to preserve its structure and function. A cell culture refers to a growth of cells in vitro ; the cells proliferate and/or differentiate but do not get organized into tissue.
The hypothalamo-neurohypophysial system secretes oxytocin and vasopressin, and shows remarkable plasticity in the adult in response to appropriate stimulation. The pituicytes are the principal cellular element of the neural lobe of the hypophysis (NH). They have the morphological characteristics of glial cells and are immunoreactive for glial fibrillary acid protein (GFAP) (Salm et al., 1982), and for the early glial markers vimentin (Marin et al., 1989) and S-100 (Cocchia and Miani, 1980).
In the resting state pituicytes surround the neurosecretory axons and terminals of the magnocellular neurons from the hypothalamus and could form a physical barrier between the blood vessels and the neurosecretory terminals. When stimulated e.g. by parturition, lactation or dehydration, an increased juxtaposition between axonal terminals and blood vessels leads to a release of neuropeptides into the general circulation. The ability of pituicytes to undergo morphological changes in response to physiological stimuli is dependent on their expression of the polysialylated neural cell adhesion molecule (PSA-NCAM) (Theodosis et al, 1991 ; Theodosis and Poulain, 1999). NH does not contain myelinated axons and thus does not contain any mature oligodendrocytes.
The applicant has identified a cell population additional to pituicytes, which express both in vitro and in vivo phenotypic markers of O-2A progenitors in the adult and developing rodent neurohypophysis. For this, the applicant has used the main following methods.

### Animals

Animal used are male rats (Sprague-Dawley) and mice (Swiss strain) which were raised in breeding colonies. Postnatal age was calculated counting postnatal (PN) day 0 as the day of birth. Rat and mouse pups from PN0 to PN3 and adult rats were used. All procedures involving the use of animals were performed in accordance with the European animal care guidelines and directives

### Antibodies

Different antibodies were used for the analysis, allowing to identify cell populations of O-2A progenitors and their progeny, and are listed below :

| Name | species, classes | Working dilution | Purchased/gifted from |
|---|---|---|---|
| GFAP | Mouse IgG | 1:4000 on section 1:500 on cells | Sigma, France |
| BrdU | Mouse IgG | 1:100 | DAKO |
| 04 | Mouse IgM | Pure supernatant | ATCC |
| Gal C | Mouse IgG | Pure supernatant | ATCC |
| Vimentin | Mouse IgM | 1:200 | Sigma, France |
| A2B5 | Mouse IgM | Pure supernatant | ATCC |
| Men B | Mouse IgM | Pure supernatant | From our laboratory |
| S-100 | rabbit | 1:300 | DAKO |
| NG2 | rabbit | 1:1000 | Chemicon |
| NG2 | Mouse IgG | 1:25 | Chemicon |
| NeuN | Mouse IgG | 1:50 | Chemicon |
| MBP | Mouse IgG | 1:50 | Euromedex |
| CNPase | Mouse IgG | 1:100 | Sigma,France |

All fluorescently labeled second antibodies were from Jackson Immunoresearch Laboratories (West Grove, PA, USA).

Glial fibrillary acidic protein (GFAP) (astrocyte cytoskeletal marker), galactocerebroside (GalC) (oligodendrocyte marker), 04 (oligodendrocyte marker) and A2B5 (oligodendrocyte surface marker) are neural cell specific surface markers able to identify the O-2A progenitors and their progeny.

### Dehydration and BrdU incorporation experiments

Adult rats were individually housed and maintained for 9 days either with normal drinking water or with 2% NaCl solution. Four injections (50 mg /kg) of BrdU (10 mg/ml in phosphate buffered saline (PBS), pH7.4) (Sigma, France) were given intraperitoneally at 12 hourly intervals before sacrifice. At least 3 control and 3 dehydrated rats were used in each experiment.
Sections, tissue pieces and explants cultures were mounted in Mowiol (Calbiotech, USA) and examined under a Zeiss Axiophot fluorescence microscope or confocal microscope.

### Statistical analysis

Proliferation was estimated by counting BrdU-positive cells on 3 to 4 randomly chosen sections for each of the 3 control and 4 dehydrated rats. Surface area was calculated using Visiolab 2000 software (Biocom) and the data were expressed as number of BrdU + cells per µm2.
The significance of the difference between control and other conditions was calculated with ANOVA using Stat View-Student software. The number of NG2-positive cells was counted on 2 randomly chosen sections from adult rat and compared to the total number of cells identified with the TOPRO3 nuclear marker.

### Immunohistochemistry and BrdU staining methods

Animals were perfused with 4 % paraformaldehyde (PF) in PBS. The pituitary glands were post-fixed for 1 hour in 4 % PF in PBS at 4°C, washed in PBS and cryoprotected in 30 % sucrose. After cryosectioning, horizontal serial sections (35 µm) were collected in PBS, incubated with primary antibodies overnight at 4°C, washed and incubated with appropriate fluorescent secondary antibodies 1 hour at RT. When necessary, permeabilization was performed for 20 minutes at RT with 0.1 % Triton-X100. For A2B5 antibody labeling, fresh whole-mount NH pieces were incubated with primary antibodies for 1 hour at room temperature. After extensive washes and 10 minutes fixation in 4 % PF in PBS, pieces were incubated with appropriate fluorescent secondary antibodies for 1hour at RT.
For BrdU labeling, preparations were incubated for 30 minutes at 37°C in 2N HCl and 0.5 % Triton-X100 for floating sections, or for 20 minutes at room temperature in 2N HCl for tissue pieces and explant cultures. After 3 washes in 0.1 M sodium tetraborate, incubation with anti-BrdU antibody was performed overnight at 4°C for floating sections or for 1 hour at room temperature for explant cultures and tissue pieces. After washes, incubation with the appropriate fluorescent secondary antibodies was performed. In the case of double staining, BrdU labeling was performed after the primary antibody incubation. When necessary, nuclear staining was performed prior to mounting using TOPRO3 (Molecular Probes, 1:1000 in PBS).

### Neurohypophysial explant cultures

Neurohypophysial culture was performed as described in Wang et al. (Wang et al., 1994) incorporated by reference. Briefly, pituitary glands from P0-P3 rat pups or adult rats (when stated) were dissected in Hanks BSS. They were stripped of meninges and the neural lobes carefully separated from the anterior and intermediate lobes. Each NH was sectioned into 4-6 pieces in Dulbecco Modified Eagle Medium (DMEM) plus 10 % fetal calf serum (FCS) medium. Pieces were then explanted on poly-L-lysine-treated glass coverslips in serum free medium (DMEM-F12 medium, supplemented with 100 µg/ml human transferin, 5 µg/ml insulin, 100 µM putrescin, 20 nM progesterone, 30 nM sodium selenite ; DMEM, GIBCO) supplemented with bFGF (10 ng/ml), PDGF-AA (10 ng/ml) and NT3 (10 ng/ml) in the presence of 0.4 % methylcellulose (Sigma, France). Cultures were incubated at 37°C in a 5 % CO2 and 95 % air atmosphere.
Labeling of in vitro explant cultures was performed after fixation for 10 minutes in 4 % PF in PBS, as described above for whole mount preparation.
Further to these practices, the invention uses, unless specifies, conventional and appropriated techniques of cell culture, cell biology, molecular biology.
A preferred embodiment for in vivo and vitro identification of O-2A progenitors will now be described.

### In vivo characterization of O-2A progenitors by brain transplantations.

Brain transplantations of newborn mouse neurohypophyses are made as follows.

Transplantations were performed as previously described (Vitry et al., 1999) incorporated by reference. Briefly, NH pieces from newborn mice were incubated in DMEM-10 %FCS containing 10 µg/ml Hoechst 33342 for 30 minutes at 37°C, washed in DMEM and injected into the brains of newborn mice, close to the subventricular zone of the left hemisphere. Mice were sacrificed at different times after transplantation (5, 15, 21 days) and brains were processed for cryostat sectioning. Serial sagital sections (14 µm) were collected and Hoechst-labeled cells were detected under UV light. Sections containing Hoechst-positive cells were used for immunofluorescence labeling as described by Vitry et al. (Vitry et al., 1999). Slides were mounted in Fluoromount and analyzed using a Wild Leitz DM fluorescence microscope.
First the applicant showed that resident O-2A progenitors exist in the adult rat NH and in the absence of cells expressing differentiated oligodendrocyte markers such as GalC. These O-2A cells are different from pituicytes.
The results are as follows. Pituicytes could clearly be identified on sections of adult rat NH as process-bearing vimentin- (Fig. 1a red) or S-100- (Fig. 1b red) positive *cells.* Cells positive for NG2, an integral membrane chondroitin sulfate proteoglycan expressed by early committed glial precursors (Nishiyama et al., 1997), were also present in the structure (Fig. 1a and b in green). Double labeling showed that the NG2+ cells (approximately 9 % of the total cell number) were vimentin- (Fig. 1a) and S-100-(Fig. 1b) negative representing a cell population distinct from the pituicytes.
To confirm unambiguously that O-2A progenitors are present in the NH of adult rats maintained under control conditions, the A2B5 antibody was used as a second marker for this population.
To avoid non-specific staining, whole-mount labeling was performed on fresh unfixed adult rat NH pieces, as described. Round bipolar or short process-bearing multipolar cells expressing the A2B5 antigen were observed in the tissue (Fig. 1c). Furthermore, immunofluorescence on sections using anti-GalC, antibody showed no labeling in the NH (not shown).
Further the applicant showed that O-2A progenitors are able to divide in vivo both during development and in the adult in response to the stimulus of dehydration.
It was first determined that dividing O-2A progenitors could be found in vivo in the NH. P3 rats were injected with BrdU and sacrificed 4 hours later. Using confocal microscopy, on whole mount preparations we clearly observed the presence of A2B5+/BrdU+ cells (Fig. 1d) in all NHs analyzed (n=3), indicating that these progenitors are present and normally divide during NH development.
Besides, in adult rat, it was determined that cell proliferation was stimulated by a dehydratation stimulus in vivo.
Cell proliferation increased in the NH, relative to paired controls, after 9 days of saline substitution of the drinking water. For these experiments, control or 9 day-dehydrated adult rats received 4 BrdU injections 2 days before being sacrificed. Immunostaining using the anti-BrdU antibody was performed on NH sections from control and dehydrated rats (Figs. 1g and 1h respectively). As described previously (Murugaiyan and Salm, 1995) the density of BrdU+ cells was significantly increased in dehydrated compared to control animals (Fig. 1i). Double-labeling was performed both on whole mount preparations and on sections with a BrdU antibody together with either an anti-A2B5 or an anti-NG2 antibody. A2B5+ (Figs. 1e and 1f) and NG2+ (Fig. 1j and k) cells were observed under both control and stimulated conditions. In dehydrated rat NHs, A2B5+/BrdU+ (Fig. 1f) or NG2+/BrdU+ (Fig. 1k) cells were clearly identified whereas A2B5+/BrdU+ cells were hardly found in the control samples (Figs. 1e and 1j).
Furthermore, a culture system was made to show that O-2A progenitors display in vitro characteristics of O-2A progenitors from other neural regions.
In vitro, characterization of O-2A progenitors from the adult rat NH was made as follows.
An in vitro assay was done using adult NH explants. BrdU was injected into control and dehydrated adult rats prior to the microdissection.
When NHs were dissected out and cultured in defined medium in the absence of any trophic factors, no cellular migration was observed around the explants (not shown). When bFGF, PDGF and NT3, known to favor migration, proliferation and survival of optic nerve O-2A progenitors (McKinnon et al., 1990 ; Barres et al., 1994) are added to the defined medium, occasional cell migration is observed after 3 days for both control and dehydrated NHs. These motile cells have a bipolar morphology, round cell bodies, two long processes (Fig. 2a) and are A2B5+ (Fig. 2b).
In control rat explants, A2B5+/BrdU+ cells are never observed among the A2B5+ migratory population (Fig. 2c). In contrast, in explants from dehydrated rats, A2B5+/BrdU+ migratory cells are observed (Fig. 2d).
Taken together the data demonstrate that resident cells exist in the adult NH, which can yield A2B5+ dividing cells after dehydration in vivo and bipolar migrating cells in vitro.
In vitro characterization of O-2A progenitors from neonatal rat NH was made as follows.
Because the adult NH explants are a poor source of migrating O-2A progenitors, characterization of these cells was also made using neonatal NH explants.
In a first set of experiments, NH explants were cultured in DMEM supplemented with 10 % FCS. An extensive emergence of fibroblasts and endothelial cells around the explant (Fig. 3a) was observed. Immunofluorescence labeling showed that the majority of cells on the top of this monolayer were GFAP+ (Fig. 3b).
In a second set of experiments, newborn NHs were cultured in defined medium supplemented with bFGF, PDGF and NT3. Fibroblasts, endothelial and bipolar cells (Wang et al., 1994) were observed among the migrating cells and A2B5 labeling was used to identify O-2A progenitors among these cells (Fig. 3c). Differentiation toward the oligodendrocyte lineage was monitored by analyzing expression of stage-specific markers. Twenty four hours after explantation, the migrating bipolar cells were both PSA-NCAM+ (Figs. 3d and 3e) and A2B5+ (Fig. 3f), whereas rare multipolar cells expressed the 04 antigen (Fig. 3g). Forty-eight hours after explantation in supplemented defined medium, PSA-NCAM+ cells were rarely found (not shown), while the majority of cells were multipolar, A2B5+ (Figs. 3h and 3i) and O4+ (Fig. 3j). After 14 days, cells had the typical morphology of mature oligodendrocytes, they expressed the 04 (Fig. 3k) and GalC (Fig. 31) antigens, and they showed MBP labeling (Fig. 3m). Under these conditions, it was not observed cells expressing a neuronal phenotype as monitored with either anti-β-III tubulin or anti-NeuN antibodies (not shown) at any of the time point examined.
Thus, O-2A progenitors from newborn rat NH are able to give rise in vitro to either mature oligodendrocytes or to astrocytes.

Further, the applicant showed the in vivo migration and differentiation potentias of newborn NH derived O-2A progenitors.
Newborn mouse NH explants were Hoechst-labeled and transplanted into the periventricular zone of newborn mouse brains as described in the methods. Three transplanted brains out of 14 were lacking Hoechst+ cells and were therefore excluded from the analysis. After different incubation period the other recipients were found to contain Hoechst+ cells (Table I) near the injection site i.e. the subventricular zone (SVZ, n=5), the dorsal lateral ventricle wall (n=2), the proximal part of the rostral migratory stream (RMS, n=1), and the corpus callosum and striatum bordering the lateral ventricle (n=2 and 1, respectively). Rare Hoechst+ cells grafted near the SVZ had migrated as far as the olfactory bulb (Fig. 4). The distribution of Hoechst+ cells in each case is summarized in Table I.

**Table I.**

| **In vivo distribution of NH-derived Hoechts+ cells after graft.** | | | | | | | | | | | |
|---|---|---|---|---|---|---|---|---|---|---|---|
| | **Wild-type NH fragments** | | | | | | | | | | |
| **Animal n°** | 1 | 2 | 3 | 4 | 5 | 6 | 7 | 8 | 9 | 10 | 11 |
| **Days post-graft** | 1 | 5 | 5 | 15 | 15 | 21 | 21 | 21 | 21 | 21 | 21 |
| **Lateral sections** | | | | | | | | | | | |
| Hippocampus | + | + | | | | | | | | | |
| Corpus callosum | | + | | | | | | | | | |
| Dorsal ventricle | + | **G** + | | | + | + | | | | **G** + | |
| Ventral ventricle | | + | | | | | | | | | |
| **Median sections** | | | | | | | | | | | |
| Hippocampus | | | ++ | + | + | | | | ++ | | |
| Fimbriae/fornix | | | ++ | + | | | | | ++ | | |
| Corpus callosum | | | | ++ | + | | **G** | | **G** | | ++ |
| Subventricular zone | **G** | | **G** | + | **G** + | **G** | | + | | | **G** + |
| Striatum | | | | | | | | **G** | | | |
| Rostral migratory stream | ++ | | | **G** + | | | | | | | + |
| Olfactory bulb | | | | + | | | | | | | + |
| *Note.* Spatiotemporal ditribution of Hoechst+ cells from newborn NH fragments after grafting near the subventricular zone of newborn mice. Grafted brains were analyzed at 1 (n=1), 5 (n=2), 15 (n=2) and 21 (n=6) days post-graft. The + and ++ symbols represent a semi-quantitative indication of the number of Hoechst+ cells found in each cerebral region. **G**, location of the graft. | | | | | | | | | | | |

Gliogenic properties of the grafted NH Hoechst+ cells, immunofluorescence analysis were performed using various markers. Hoechst+ cells were found which expressed CNPase (Figs. 5a-5d) or GFAP (Figs. 5e-5h) in various cerebral locations.
This result demonstrates that cells from the NH are able to give rise in vivo to both astrocytes and mature oligodendrocytes.
Very surprisingly, using an anti-NeuN antibody (a specific neuronal marker) it was shown that some Hoechst+ cells also differentiated as neurons. These cells were localized in the neurogenic cerebral cortex and sometime in the corpus callosum migrating from the lateral wall to the cortex (Figs. 5i-5l). Thus NHs grafted in newborn mouse brain could generate neurons in the complete absence of neuronal progenitors. Heterotypic transplantations of the neurohypophysis into neonatal brain revealed the presence of pluripotent cells able to generate neurons as well as astrocytes and oligodendrocytes. It is not clear whether these neurons are derived from O-2A progenitors or from the pituycites but in any case, the transplantation of the pieces of isolated neurohypophysis is efficient to generate neurons in vivo.
According to the inventors, both the neuroectodermal and focal origin of the NH is consistent with the presence of O-2A progenitors in this structure. Furthermore, dividing O-2A progenitors are present in neonatal rats while the neural lobe is still developing, suggesting that these cells participate in the formation of the gland. Both the present studies in vivo and in vitro show that O-2A progenitors are resident cells of the NH, strongly suggesting that they could give rise to the major cell type of this structure, the pituicyte. In support, the applicant has shown that the NH does not contain oligodendrocyte lineage cells other than the A2B5+/NG2+ O-2A progenitors. Moreover, in the presence of serum, these cells differentiate into GFAP+ cells, a characteristic of pituicyte. Interestingly, it was observed that, O-2A progenitors from the perinatal structure divide faster than those of adult NH.
After having characterized O-2A progenitors in NH in vivo and in vitro, a few examples of applications of such presence to neurobiology and therapy will now be described. By using the methods presented above, the present invention can provide cellular populations of O-2A progenitors isolated from NH which may be cultured in appropriate conditions to regenerate and differenciate into namely oligodendrocytes, astrocytes. This culture will be made by appropriated, for instance described in US 5,693,482, the invention allowing to use a new source of O-2A progenitors, meaning the NH. For instance the culture medium may be as follows.
Basal medium consists in Dubelco Modified Eagle Medium (DMEM)/F12 (50/50), supplemented with 100 µg/ml human transferring, 5 µg/ml insulin, 100 µM putrescin, 20 nM progesterone, 30 nM sodium selenite ; (GIBCO, BRL). This medium is supplemented with bFGF (10 ng/ml), PDGF-AA (10 ng/ml) and NT3 (10 ng/ml) for the maintenance of O-2A progenitors in culture. In order to differentiate the O-2A progenitors in astrocytes the basal medium is supplemented with 15 % fetal calf serum (FCS) and PDGF-AA (10ng/ml). Differentiation of the 0-2A progenitors in oligodendrocytes is obtained in basam medium. Cultures are kept ay 37°C in a 5 % CO2 and 95 % air atmosphere.
The invention is illustrated using O-2A progenitors issued from the rat. However O2-A progenitors and their progeny may be isolated from NH from human and non-human primates, equines, canines, felines, bovines, porcines, etc.
The invention provides cellular preparations comprising an enriched population of O-2A progenitors or their progeny obtained from the culture, differentiation and isolation of O-2A progenitors in appropriate medium. The preparation obtained contains a majority of or at least about 75 % of the population selected, and preferably 90 to 95 %.
It is reminded that in order to isolate progenitor cells from the neurohypophysis explant, an agent which causes proliferation of the progenitor cells is useful. Different techniques may be used to assess the proliferation (such as DNA synthesis measuring, morphological changes), and to isolate the progenitor cells having proliferated in an explant (such as mechanical isolation, enzymatic digestion of the explant followed by the isolation of the activated progenitor cell population based on specific cell surface markers). Once the different cell populations originated from O-2A progenitors have been identified (using monoclonal antibodies able to identify surface markers associated to specific stage of differentiation), procedure for separation may use magnetic separation, chromatography, fluorescence activated cell sorting, direct separation using markers such as magnetic beads reacting with a support.
It may further be very useful to prepare genetically-engineered mammalian multipotent O-2A progenitors and their progeny, which are cultured in order to grow a sufficient number of cells for in vitro gene transfer followed by in vivo implantation. Nucleic acid sequences encoding genes of interest are introduced into multipotent O-2A progenitors where they are expressed. These genes can include neurotrophic or survival factors, immortalizing oncogenes, marker genes.
The O-2A progenitors may be immortalized to maintain the cell at a defined developmental stage. The present techniques for immortalization typically involve the transfection of an oncogene to the cell. Transfection of the oncogene can be accomplished by appropriated, including using recombinant retroviruses, chemical or physical methods (calcium phosphate calcium-phosphate-mediated transfection, microinjection, insertion of a plasmid present in liposomes).
For example, one method is to use an eukaryotic viral vector, such as simian virus 40 (SV40) or bovine papilloma virus, to transiently infect or transform the O-2A progenitors.
Various viral vectors can be utilized for immortalization including adenovirus, adenoassociated virus, herpes virus, vaccinia, retrovirus. Examples of retroviral vectors in which a single foreign gene can be inserted include Moloney murine, leukemia virus (MoMuLV), Harvey murine sarcoma virus (HaMuSV), murine mammary tumor virus (MuMTV), gibbon ape leukemia virus (GaLV) and Rous Sarcoma Virus (RSV). A number of additional retroviral vectors can incorporate multiple genes. All of these vectors can transfer or incorporate a gene for a selectable marker so that transduced cells can be identified and generated.
Herpes virus-based vectors may also be used to transfer genes into a O-2A progenitors : herpes viruses are capable of establishing a latent infection and an apparently non-pathogenic relationship with some neural cells, such vectorbased on HSV-1, for example, may be used. Similarly, it may be possible to use human and animal viruses that infect cells of the CNS efficiently, such as rabies virus, measles, and other paramyxoviruses and even the human immunodeficiency retrovirus (HIV), to develop useful delivery and expression vectors.
When a recombinant retrovirus is engineered to contain an immortalizing oncogene, the oncogene can be any one of those known to immortalize. For example, such commonly used immortalizing genes include genes of the myc family (c-myc and v-myc), adenovirus genes (E1a 12s and E1a 13s), the polyoma large T antigen and SV40 large T antigen.
Marker genes, such as the E.coli β.galactosidase gene, can be introduced into O-2A progenitors allowing the identification of these cells and their progeny. Selectable marker genes, such as the neomycin phosphoribosyltransferase (neomycin-resistance) may be introduced to provide for a population of genetically-engineered O-2A progenitors which are identified by the ability to grow in the presence of selective pressure (i. e. medium containing neomycin).
Numerous useful genes may be introduced into O-2A progenitors and their progeny for identifying drugs, in particular using genes for a receptor molecule. For example, such neuronal receptors include the receptor which binds dopamine, GABA, adrenaline, noradrenaline, serotonin, glutamate, acetylcholine and various other neuropeptides. Transfer and expression of a particular receptor in O-2A progenitors of specific neural origin, would allow identification of neuroactive drugs and trophic factors which may be useful for the treatment of diseases involving that O-2A progenitors type and that receptor. For example, a neuroactive compound which mimics a neurotransmitter and binds to a receptor and exhibits either an antagonistic or agonist effect, thereby inhibiting or stimulating a response in O-2A progenitors, can be identified.
The introduction in a patient treated of O-2A progenitors and their progeny incorporating a gene unsufficiently expressed by the patient may be useful. For instance a gene encoding a neurotrophic factor, such as nerve growth factor, (NGF), would prevent from a degeneration of cholinergic neurons, in particular for treatment of Alzheimer's disease which is characterized by degeneration of the cholinergic neurons of the basal forebrain. A gene encoding L-DOPA, the precursor to dopamine, would be useful for treatment of Parkinson's disease which is characterized by a loss of dopamine neurons in the substantia-nigra of the midbrain.
The introduced gene may also be a gene capable of inhibating factors responsible for cell death in certain areas, for example in case of a stroke, or a gene efficient against a tumor.
The present invention also provides a method of treating a subject with a cell disorder of the CNS which comprises administering to the subject a therapeutically effective amount of the O-2A progenitors or their progeny.
The method of treating a subject with a CNS disorder comprises intracerebral grafting of O-2A progenitors, or oligodendrocytes or astrocytes or neurons which have been induced to differentiate from the O-2A progenitors, to the region of the CNS having the disorder.
O-2A progenitors graft involves typically transplantation of cells into the CNS or into the ventricular cavities or subdurally onto the surface of a host brain. Such methods for grafting are described in Neural Grafting in the Mammalian CNS, Bjorklund and Stenevi, eds., (1985), incorporated by reference herein. Procedures include in particular intraparenchymal transplantation, (i.e., within the host brain) achieved by injection or deposition of tissue within the host brain so as to be apposed to the brain parenchyma at the time of transplantation.
Administration of the O-2A progenitors into selected regions of the recipient subject's brain may be made by :
- drilling a hole and piercing the dura to permit the needle of a microsyringe to be inserted ;
- injecting intrathecally into the spinal cord region O-2A progenitors or progeny preparation allowing to graft O-2A progenitors or progeny to any predetermined site in the brain or spinal cord.
Multiple grafting can be made simultaneously in several different sites using the same cell suspension or from different anatomical regions : for example, the O-2A progenitors or progeny (oligodendrocytes or astrocytes) may be grafted into the CNS of a subject with multiple sclerosis, wherein the subject's oligodendrocytes have died.
The method of treating a subject with a CNS disorder also contemplates the grafting of O-2A progenitors in combination with other therapeutic procedures useful in the treatment of disorders of the CNS. For example, the O-2A progenitors can be coadministered with agents such as growth factors, gangliosides, antibiotics, neurotransmitters, neurohormones, toxins, neurite promoting molecules and antimetabolites and precursors of these molecules such as the precursor of dopamine, L-DOPA.
Concerning neurons very surprisingly found to differentiate after a graft of NH explant, the grafting from NH explant can now be made as described herein. The grafting may be done between species, for instance a graft of porcine neurons to human.
The invention also provides a method of identifying compositions which affect O-2A progenitors or progeny, such as by inhibiting or stimulating O-2A progenitors to proliferate or differentiate into oligodendrocytes, astrocytes, neurons.

### REFERENCES

Barres BA, Raff MC, Gaese F, Bartke I, Dechant G, Barde YA (1994) A crucial role for neurotrophin-3 in oligodendrocyte development. Nature 367:371-375.

Butt MA (1998) Macroglial cell types, lineage, and morphology in the CNS. Ann N Y Acad Sci 1991:633.

Cocchia D, Miani N (1980) Immunocytochemical localization of the brain-specific S-100 protein in the pituitary gland of adult rat. J Neurocytol 9:771-782.

Kondo T, Raff M (2000) Oligodendrocyte precursor cells reprogrammed to become multipotential CNS stem cells. Science 289:1754-1757.

Levine JM, Stallcup WB (1987) Plasticity of developing cerebellar cells in vitro studied with antibodies against the NG2 antigen. J Neurosci 1987 ,7(9):2721-31.

Marin F, Boya J, Lopez-Carbonell A (1989) Immunocytochemical localization of vimentin in the posterior lobe of the cat, rabbit and rat pituitary glands. Acta Anat (Basel) 134:184-190.

McKinnon RD, Matsui T, Dubois-Dalcq M, Aaronson SA (1990) FGF modulates the PDGF-driven pathway of oligodendrocyte development. Neuron 5:603-614.

Raff MC, Miller RH, Noble M (1983) A glial progenitor cell that develops in vitro into an astrocyte or an oligodendrocyte depending on culture medium. Nature 303:390-396.

Salm AK, Hatton GI, Nilaver G (1982) Immunoreactive glial fibrillary acidic protein in pituicytes of the rat neurohypophysis. Brain Res 236:471-476.

Theodosis DT, Bonhomme R, Vitiello S, Rougon G, Poulain DA (1999) Cell surface expression of polysialic acid on NCAM is a prerequisite for activity-dependent morphological neuronal and glial plasticity. J Neurosci 19:10228-10236.

Theodosis DT, Poulain DA (1999) Contribution of astrocytes to activity-dependent structural plasticity in the adult brain. Adv Exp Med Biol 468:175-182.

Theodosis DT, Rougon G, Poulain DA (1991) Retention of embryonic features by an adult neuronal system capable of plasticity: polysialylated neural cell adhesion molecule in the hypothalamo-neurohypophysial system. Proc Natl Acad Sci U S A 88:5494-5498.

Vitry S, Avellana-Adalid V, Hardy R, Lachapelle F, Baron-Van Evercooren A (1999) Mouse oligospheres: from pre-progenitors to functional oligodendrocytes. J Neurosci Res 58:735-751.

Wang C, Rougon G, Kiss JZ (1994) Requirement of polysialic acid for the migration of the O-2A glial progenitor cell from neurohypophyseal explants. J Neurosci 14:4446-4457.

## Claims

1. An isolated population of mammalian neurohypophysis cells comprising multipotent cells.

2. A population according to claim 1 wherein the multipotent cells are able to differentiate into at least oligodendrocytes and/or type II astrocytes and/or neurons.

3. A population according to claim 1 or 2, wherein said population is derived from newborn neurohypophysis when this structure is still developing or from adult neurohypophysis.

4. A population according to anyone of claims 1 to 3, wherein said population is derived from adult neurohypophysis after a physiological stimulus.

5. A population according to claim anyone of claims 1 to 4, wherein said population is an explant of neurohypophysis, cultivated in an appropriate culture medium.

6. A method for obtaining a cellular population according to anyone of claims 1 to 5, said method comprising the steps of preparing a suspension from a neurohypophysis explant, culturing the suspension in an appropriate medium for growth and/or proliferation of said population.

7. A method for isolating a cellular population according to anyone of claims 1 to 5 comprising
- culturing a neurohypophyse explant wherein said explant is maintainable in culture and includes progenitor cells that have the ability to differentiate into at least oligodendrocytes and/or type II astrocytes and/or neurons ;
- contacting said explant with an agent which causes proliferation of progenitor cells of said explant;
- isolating from said explant progenitor cells that proliferate in response to said agent.

8. A method for obtaining oligodendrocyte-type II astrocytes (O-2A) progenitor cells and/or their progeny comprising
- culturing a neurohypophysis explant wherein said explant is maintainable in culture and includes progenitor cells that have the ability to differentiate ;
- contacting said explant with an agent which causes proliferation of progenitor cells of said explant and differentiation into oligodendrocyte-type 2 and/or astrocyte ;
- contacting said suspension with markers for O-2A progenitor cells and/or their progeny;
- isolating O-2A progenitor cells and/or their progeny.

9. A method according to claim 9, wherein the isolation technique of O-2A progenitor cells and/or their progeny includes one of magnetic separation, antibody coated magnetic beads, affinity chromatography, antibodies attached to a matrix, responsiveness to growth factors, specific gene expression, antigenic cell specific surface markers, basic morphology.

10. A method according to claim 9 or 10, further comprising the preparation of an isolated cellular composition containg at least 50 %, of neurohypopysis O-2A progenitor cells.

11. A method for screening compounds having an ability to modulate one of growth, proliferation and/or differentiation of progenitor cells obtained from neurohypophysis comprising :
- culturing a neurohypophysis explant wherein said explant is maintainable in culture and includes progenitor cells that have the ability to differentiate ;
- contacting said explant with an agent which causes proliferation of progenitor cells of said explant;
- contacting said explant with a tested compound ;
- detecting one of growth, proliferation and/or differentiation of progenitor cells by comparing the results without the compound.

12. A method for obtaining an isolated population of transformed mammalian multipotent oligodendrocytes-type 2 astrocytes (O-2A) progenitor cells and/or their progeny, said method comprising the introduction of at least a nucleic acid.

13. A population of transformed mammalian multipotent oligodendrocytes-type 2 astrocytes (O-2A) progenitor cells and/or their progeny obtainable according to the method of claim 12.

14. A method for providing neurophypophysis O-2A progenitor cells and/or their progeny in at least one location of the brain, comprising the implantation of O-2A progenitor cells in the brain.

15. A method according to claim 14 wherein the O-2A progenitor cells have been prior transformed.

16. A method to assay in vivo development and differentiation of a neurohypophysis explants containing O-2A progenitor cells , said method comprising
- extracting a newborn or adult neurohypophysis explant;
- labeling and transplantating the explant in the periventricular zone of newborn or adult mammalian brain;
- identifying the distribution of transplanted cells in the brain.

17. A method for screening antibodies capable of recognizing surface markers which characterizes multipotent O-2A progenitor cells and/or their progeny comprising culturing said cells in an appropriate medium, adding tested antibodies, identifying the complex antibodies-markers.

18. A method for providing antibodies capable of recognizing surface markers which characterizes multipotent O-2A progenitor cells and/or their progeny comprising immunizing an animal with said cells, isolating the antibodies produced.

19. An isolated mammalian progenitor cell wherein said progenitor cell is extracted from neurohypophysis and is able to differentiate into at least oligodendrocyte and/or type II astrocyte and/or neuron.

20. A method for obtaining at least one isolated cell according to claim 18 comprising
- culturing a neurohypophysis explant wherein said explant is maintainable in culture and includes progenitor cells that have the ability to differentiate into at least oligodendrocytes and type II astrocytes ;
- isolating from said explant at least one progenitor cell that have the ability to differentiate into at least oligodendrocytes and type II astrocytes.

21. A pharmaceutical composition comprising a population of neurohypophysis O-2A progenitor cells and/or their progeny, or comprising said population that has been transformed, and a vehicle pharmaceutically acceptable.

22. A composition according to claim 21 comprising, as a cellular population, at least 50 % of the O-2A progenitor cells and/or their progeny.

23. A composition according to claim 21 or 22, used as a medicament for a neural disorder or a neural disease.

24. Use of a population obtained by the method according to anyone of claims 6 to 12, for the preparation of a pharmaceutical composition for treatment of a neural disorder or a neural disease.

25. Use of an explant according to claim 5 for the preparation of a graft for treatment of a neural disorder or a neural disease.

26. Use according to claim 24 or 25 wherein the neural disease is Alzheimer's disease, Parkinson's disease.
